# EUROPEAN PATENT APPLICATION

(11) **EP 1 734 130 A1**
(43) Date of publication of application: **20.12.2006**
(21) Application number: 05076396.0
(22) Date of filing: 16.06.2005
(51) Int. Cl.: C12P 7/42

(54) **Improved biosynthetic production of 2,3-trans-CHD**

(71) Applicant: DSM IP Assets B.V., 6411 TE Heerlen (NL); Forschungszentrum Jülich GmbH, 52425 Jülich (DE); DSM Biotech GmbH, 40474 Düsseldorf (DE)
(72) Inventor: Bujnicki, Robert Piotr, 52445 Titz-Rödingen (DE); Bongaerts, Johannes Josef, 51379 Leverkusen (DE); Raeven, Leon Jean Renier Marie, 6132 HS Sittard (NL); Sprenger, Georg, 71522 Backnan-Maubach (DE); Kuhm, Andrea, 71069 Sindelfingen (DE); Takors, Ralf, 33334 Guetersloh-Isselhorst (DE)
(74) Representative: Verhaegen, Ilse Maria M.

(57) **Abstract**

The invention relates to a process for the biosynthetic production of [5*S*,6*S*]-5,6-dihydroxy-cyclohexa-1,3-diene-1-carboxylic acid (2,3-*trans*-CHD) in an *Escherichia* microorganism having increased activity of isochorismate synthase and isochorismatase, and being deficient in activity of 2,3-dihydroxybenzoate synthase, wherein the process is carried out in a chorismate overproducing host microorganism also having decreased glucose dehydrogenase activity. In this improved biosynthetic process, 2,3-*trans*-CHD is being produced in high yields and at concentrations in the biosynthetic reaction medium of at least 12 g/l.

The invention further relates to the use of the supernatant of a process for the biosynthetic production of [5S,6S]-5,6-dihydroxy-cyclohexa-1,3-diene-1-carboxylic acid (2,3-trans-CHD) where the concentration of 2,3-*trans*-CHD in the process has arrived at a concentration of at least 12 g/l as a source for a co-factor for PQQ dependent enzymes.

## Description

The present invention relates to an improved process for the biosynthetic production of [5S,6S]-5,6-dihydroxycyclohexa-1,3-diene-1-carboxylic acid (2,3-*trans*-CHD) in an *Escherichia* microorganism having increased activity of isochorismate synthase and isochorismatase, and being deficient in activity of 2,3-dihydroxybenzoate synthase. Biosynthetic syntheses, in general, are processes wherein at least one enzymatic reaction step is taking place. When such processes occur in living microorganisms, the process is called an *"in vivo"* process. As meant herein, the term "increased activity" (of an enzyme) means that the level of a specific enzyme activity is increased to a level of activity above the level of such activity naturally existing in said microorganism under identical conditions. It will be understood that such increased level of enzyme activity can be achieved by all means known to the skilled man, for instance by overexpression of the corresponding gene.

Another name for 2,3-*trans*-CHD is (S,S)-2,3-dihydroxy-2,3-dihydrobenzoic acid. 2,3-*trans*-CHD is an intermediate in the biosynthetic pathway to enterobactin (for instance in *Escherichia coli*), and can be used as a building block for the synthesis of a large variety of other molecules due to the fact that the 2,3-*trans*-CHD molecule contains two chiral C-atoms, two double bonds, two hydroxyl groups and a carboxylic group.

2,3-*trans*-CHD is one of the metabolic pathway products derived from chorismate via the intermediacy of isochorismate. Biosynthetic production of 2,3-*trans*-CHD has been described in WO 02/18611 where this biosynthesis (*in casu* a fermentation process) occurs in *Escherichia coli* strains that are deficient in 2,3-dihydroxybenzoate synthase (an enzyme coded for by the gene *entA*). It is shown in WO 02/18611, that 2,3-*trans*-CHD can be produced in amounts of up to about 4,6 g/l. The transformation from chorismate into 2,3-*trans*-CHD occurs by means of two enzymes: isochorismate synthase (coded by the gene *entC*) and isochorismatase (coded by the gene *entB*).

The known process for the biosynthetic production of 2,3-*trans*-CHD as described in WO 02/18611, at the 2,3-*trans*-CHD concentration levels obtained therein, does not pose real problems, except for the fact that the titers obtained in this process are considered to be still quite low if a commercially attractive process is envisaged. Accordingly, there is need for finding possibilities for increasing the titer and yield of 2,3-*trans*-CHD in the biosynthetic production process.

It has been demonstrated in further, but yet unpublished, studies of the present inventors regarding the biosynthetic production of 2,3-*trans-*CHD, that the yields of 2,3-*trans*-CHD, as achievable by the methods of WO 02/18611, indeed could be further improved substantially by performing the biosynthesis in *Escherichia strains,* more particularly in *Escherichia coli* strains, with additionally overexpressed *aroFBL* genes. This indeed resulted in a strong increase of the concentration level of 2,3-*trans*-CHD, but simultaneously the disadvantage was found that production of 2,3-*trans*-CHD at relatively high concentrations (i.e. when concentration of 2,3-*trans*-CHD has reached about at least 12 g/l) is accompanied by strong gluconate by-product formation from the glucose used as carbon source for the fermentation, which by-product formation increases as concentration of 2,3-*trans*-CHD becomes higher. Evidently, such by-product formation lowers the yield of 2,3-*trans*-CHD on glucose consumed. For further use of 2,3-*trans*-CHD in the production of derivatives thereof it is, moreover, highly disadvantageous if the 2,3-*trans*-CHD is contaminated with gluconate.

Purifying 2,3-*trans*-CHD from the by-product gluconate is troublesome and would require, for instance, repeated recrystallisations, because other types of purification processes (e.g. anionic adsorption, reactive extraction or electrodialysis) would have quite indiscriminating affinity for both the gluconate by-product and the desired product 2,3-*trans*-CHD. Accordingly, and this is the aim of the present invention, there is need for finding possibilities for increasing the titer and yield of 2,3-*trans*-CHD in the biosynthetic production process whilst avoiding gluconate by-product formation and leading to 2,3-*trans*-CHD substantially free of gluconate, so that no such further purification steps are required.

As meant herein, the term "substantially free of gluconate" means that the gluconate concentration in the fermentation supernatant will be at most 1 g/l, preferably at most 0,5 g/l. and most preferably at most 0,1 g/l.

The present invention also relates to a source for an alternative (and thusfar unknown) co-factor for PQQ-dependent enzymes (pyrroloquinoline quinone dependent enzymes).

The present inventors now surprisingly have found an improved process for the biosynthetic production of [5S,6S]-5,6-dihydroxy-cydohexa-1,3-diene-1-carboxylic acid (2,3-*trans*-CHD) in an *Escherichia* microorganism having increased activity of isochorismate synthase and isochorismatase, and being deficient in activity of 2,3-dihydroxybenzoate synthase, wherein the process is carried out in a chorismate overproducing host microorganism also having decreased glucose dehydrogenase activity.

The 2,3-*trans*-CHD obtained by the improved, high yield (i.e. at a titer higher than about 10-12 g/l), process according to the invention is substantially free of gluconate.

It is first of all to be noted, that it is completely unexpected that 2,3-*trans-*CHD biosynthesis is accompanied by gluconate formation. Gluconate formation in microorganisms such as *E. coli* is known to occur (periplasmically) under the influence of a membrane bound, and PQQ (pyrroloquinoline quinone) dependent, glucose dehydrogenase enzyme (Gdh), but only if PQQ is added. This can be seen from a number of references: for instance, Hommes, R.W. et al., FEMS Microbiol. Lett., Vol.24, p.329-333, 1984; Ameyama, M. et al., Agric. Biol. Chem., Vol.50, p.49-57, 1986; Hommes, R.W. et al., Antonie van Leeuwenhoek, Vol.60, p.373-382, 1991. From the said literature it would follow that, without addition of PQQ, no gluconate formation takes place from glucose in *E*. *coli* cells. Goldstein, A. et al., Biochim. Biophys. Acta, 1647, p.266-271 (2003), however, suggests that inducible PQQ biosynthesis might be taking place, the induction being caused by expression of heterologous genes. In the process for the biosynthesis of 2,3-*trans*-CHD according to the present invention no PQQ is being added at all, nor have any heterologous genes been expressed. Nevertheless, gluconate formation is observed in biosynthesis of 2,3-*trans*-CHD at high titers thereof, if the process is not carried out according to the process of the present invention. Such strong formation of gluconate is not only unexpected, but also undesirable. Increased levels of gluconate in the fermentation broth are, for instance, likely to be causing osmotic stress for the host cells, and thereby have a negative effect on growth and/or product formation, and thus also on the maximum obtainable amount of 2,3-*trans*-CHD to be formed.

According to the (*in vivo*) biosynthetic process of the present invention not only very high titers of 2,3-*trans*-CHD are achieved (of about up to 20 g/l), but also at high conversion yields from glucose and without substantial gluconate by-product formation. It is thereby avoided that glucose import, by means of the microorganism's phosphotransferase system (PTS system), into the cells is lowered because of the periplasmatic conversion into gluconate, that unexpectedly occurs without addition of PQQ and without expression of heterologous genes.

Chorismate producing microorganisms generally are microorganisms wherein first shikimate is being formed via any of the known shikimate biosynthetic pathways. Further conversion of shikimate formed into chorismate takes place by means of phosphorylation, addition of a further pyruvate group and dephosphorylation. In fact, this full biosynthetic reaction sequence forms part of the aromatic amino acid pathway.

Chorismate overproducing host organisms are host organisms that, due to genetic modification of their basic metabolism, are capable of giving increased biosynthetic production of chorismate. Such host organisms, for instance (and preferably) have been engineered by a combination of the following measures: deletion of the *tyrA* and *pheA* genes, insertion of the *aroF*^{for} gene (feed-back-resistant DAHP), and/or overexpression of *aroFBL* genes. The skilled man can easily assess whether a host strain is a suitable chorismate overproducer, or not.

Glucose dehydrogenase activity is the enzymatic activity required for the conversion of glucose into gluconate. As mentioned above, in *Escherichia* cells this enzymatic conversion requires PQQ (pyrroloquinoline quinone) as co-factor. Decreased glucose dehydrogenase activity means that ― when PQQ is being added - the (modified, decreased) glucose dehydrogenase activity is lower than the activity of the said (unmodified) glucose dehydrogenase in its natural surroundings when PQQ is being added. The enzyme causing glucose dehydrogenase activity in *Escherichia* is the so-called Gdh protein. It is coded for by the gcd gene. More particularly, the glucose dehydrogenase gene gcd is coding for the apoenzyme of the PQQ dependent glucose dehydrogenase enzyme. Accordingly, the term gcd gene is intended to encompass any gene coding for the function (and/or the apoenzyme) of a PQQ dependent glucose dehydrogenase enzyme.

Determining the glucose dehydrogenase activity (and assessing whether glucose dehydrogenase activity is decreased or not) can be done as described by Velterop et al., Journal of Bacteriology Vol.177, p. 5088-5098 (1995), or likewise by Cozier et al., Biochemical Journal, Vol.340, p.639-647 (1999).

Preferably, the decreased glucose dehydrogenase activity in the *Escherichia* microorganism has been achieved by lowering the expression of the glucose dehydrogenase gene gcd in the said microorganism.

Alternatively, and deemed to be encompassed by the general term of "lowering the expression" (i.e. leading to "less active glucose dehydrogenase enzymes"), also mutant gcd genes can be applied that, for instance, result in providing enzymes that are substituted near the active center by one or more bulky, or positively or negatively charged, groups and that thereby have strongly lowered or even totally switched-off glucose dehydrogenase activity.

It is especially preferred that the decreased glucose dehydrogenase activity in the *Escherichia* microorganism has been achieved by deletion of the glucose dehydrogenase gene gcd from the said microorganism, or by switching-off the activation of said gene by any means known to the skilled man, so as to obtain a gcd negative (*gcd⁻*) microorganism.

As meant herein the term "*gcd* negative" (*gcd⁻*) microorganism indicates that there is no expression of the gcd gene in the microorganism. As a result the activity level of the glucose dehydrogenase enzyme in the microorganism, even when PQQ is being added, is about zero, and is thus very much lower than the activity level of said enzyme naturally existing in said microorganism under identical conditions.

Deletion (disruption) of the glucose dehydrogenase gene gcd from the microorganism can be done very efficiently by the method described by Datsenko, K.A. and Wanner, B.L, Proc. Natl. Acad. Sci. USA, 97, p. 6640-6645, 2000, followed by subsequent deletion of the antibiotic resistance cassette.

In the biosynthetic process according to the invention, the increased activity of isochorismate synthase and isochorismatase is preferably obtained by overexpression of the genes *entB* and *entC* and/or overexpression of the genes *entB* and *menF* from an *Escherichia* strain, preferably from an *Escherichia coli* strain.

It is especially preferred that the one or more overexpressed genes from the group of *aroFBL* genes, and/or the overexpressed genes *entB* and *entC* and/or the overexpressed genes *entB* and *menF,* are under control of an inducible promoter, for instance, of the IPTG-inducible promoter Ptac. However, any further known genetic measure or measures, that can be applied singly or in combination for increasing the carbon flux into and through the aromatic biosynthesis pathway, can suitably applied for achieving further improvement of the process according to the present invention.

Preferably, the *Escherichia* microorganism contains one or more overexpressed genes from the group of *aroFBL* genes from an *Escherichia* strain.

The one or more overexpressed genes from the group of *aroFBL* genes are obtained by overexpression of such genes from an *Escherichia* strain, preferably from an *Escherichia coli* strain. Accordingly, these overexpressed genes are homologous to the host strains used in the process according to the present invention.

Preferably, the *Escherichia* microorganism used as host microorganism in the process of the present invention is an *Escherichia coli* microorganism.

Recovery of the [5S,6S]-5,6-dihydroxy-cyclohexa-1,3-diene-1-carboxylic acid (2,3-*trans*-CHD) formed in the process of the present invention can be carried out by any means known to the skilled man. Most preferably, however, the [5*S*,6*S*]-5,6-dihydroxy-cyclohexa-1,3-diene-1-carboxylic acid (2,3-*trans*-CHD) formed in the process is separated from the microorganism biomass by *in situ* product recovery (ISPR). Suitable methods for ISPR are described in, for instance, EP-B-1181089 (using membranes) or in WO-03/092853 (using centrifuges).

Without being bound to any scientific theory or concept, the present inventors conclude from their observations in the improved biosynthesis of [5S,6S]-5,6-dihydroxy-cyclohexa-1,3-diene-1-carboxylic acid (2,3-*trans*-CHD) according to the present invention, that the supernatant of this biosynthetic process surprisingly contains one or more products, other than PQQ, that can act as co-factor for PQQ-dependent enzymes. Accordingly, a further embodiment of the present invention is based on this observation, which can find important industrial use in all kinds of processes where PQQ dependent enzymes are involved. This embodiment of the present invention, thus, relates to a source for an alternative (and thus far unknown) co-factor for PQQ-dependent enzymes.

In particular, this embodiment of the present invention relates to the use of the supernatant of a process for the biosynthetic production of [5*S*,6*S*]-5,6-dihydroxy-cyclohexa-1,3-diene-1-carboxylic acid (2,3-*trans*-CHD) where the concentration of 2,3-*trans*-CHD in the process has arrived at a concentration of at least 12 g/l as a source for a co-factor for PQQ dependent enzymes.

The invention is further elucidated by means of the following examples and comparative examples. The examples, however, are by no means intended to restrict the scope of the claims.

### Experimental part

### General procedures for Examples 1 and 2

Standard molecular cloning techniques such as plasmid DNA isolation, gel electrophoresis, enzymatic restriction modification of nucleic acids, *E. coli* transformation etc. were performed as described by Sambrook et al., 1989, "Molecular Cloning: a laboratory manual", Cold spring Harbor Laboratories, Cold Spring Harbor, NY. Synthetic oligonucleotides were obtained from MWG-Biotech AG (Ebersberg, Germany). DNA sequence analyses were performed by GATC Biotech AG (Konstanz, Germany) and AGOWA GmbH (Berlin, Germany).

### Example 1: Construction of plasmid pC20

As expression vector for the 2,3-CHD biosynthesis genes, plasmid pJF119EH was chosen (Fürste, et al. (1986) Gene, 48:119-131). The expression system uses the IPTG inducible *tac* promoter and carries the *lac* repressor (*lac*l^{q} gene), which keeps the expression of the cloned foreign gene in the absence of the inducer extremely low.

Genomic DNA was prepared from *E*. *coli* W3110 strain LJ110 (Zeppenfeld et al. (2000) J.Bacteriol., 182: 4443-4452). A 1213 bp DNA-fragment comprising the gene *aroF* for the DAHP synthase (tyr) was amplified by PCR from *E. coli* LJ110 chromosomal DNA (nucleotides 5872-6942 of accession number AE000346; amplified region nucleotides 5786-6965) using the following primers:
5'- CTATCGAATTCGAGCATAAACAGGATCGCC - 3' [SEQ ID: No. 1]
   (with *Eco*RI recognition site underlined) and
5'- CACTTCAGCAACCAGTTCCCGGGGCTTCGC - 3' [SEQ ID: No. 2]
   (with Smal recognition site underlined).

A list of all nucleotide sequences used in the context of the present application is presented in the SEQUENCE LISTING annexed hereto.

Correct size of the amplified DNA-fragment was confirmed by agarose gel electrophoresis and the DNA-fragment was purified from the gel. The DNA-fragment and the plasmid pJF119EH were restricted with the restriction enzymes *Eco*Rl and Smal. The two DNA-fragments were subsequently ligated and used for the transformation of chemically competent cells of *E. coli* DH5α (Invitrogen GmbH, Karlsruhe, Germany). The transformants were selected on LB agar plates containing 100 mg·l⁻¹ ampicillin. A plasmid showing the correct insert sequence (as confirmed by sequencing) was called pJF119EH-*aroF.*

A 1151 bp DNA-fragment comprising the gene *aroB* for the 3-dehydroquinate synthase was amplified by PCR from *E. coli* LJ110 chromosomal DNA (nucleotides 6410-7498 of accession number AE000414; amplified region nucleotides 6402-7521) using the following primers:
5'-ACTCGTCTGCGGGATCCGTAATTAAGGTGG - 3' [SEQ ID: No. 3]
   (with *Bam*HI recognition site underlined) and
5'-AGCGGCCTAAGCTTTCTTGTTGTTACGCTG - 3' [SEQ ID: No. 4]
   (with *Hin*dIII recognition site underlined).

Correct size of the amplified DNA-fragment was confirmed by agarose gel electrophoresis and the DNA-fragment was purified from the gel. The DNA-fragment and the plasmid pJF119EH-*aroF* were restricted with the restriction enzymes *Bam*HI and *Hin*dIII. The two DNA-fragments were ligated and used for the transformation of chemically competent cells of *E*. *coli* DH5α.

Transformants were selected on LB agar plates containing 100 mg·l⁻¹ ampicillin. A plasmid showing the correct insert sequence (as confirmed by sequencing) was called pJF119EH-*aroF-aroB.*

A 600 bp DNA-fragment comprising the gene *aroL* for the shikimate kinase II was amplified by PCR from *E*. *coli* LJ110 chromosomal DNA (nucleotides 5281-5805 of accession number AE000145; amplified region nucleotides 5262-5816) using the following primers:
5' - CATGACACCGGCTTTCGCCGCATAAGCTTCTATTGG - 3' [SEQ ID: No. 5]
   (with *Hind*III recognition site underlined) and
5' - TGAACGTTAAAAGCTTGCGCTCGAAAATCAACAATT - 3' [SEQ ID: No. 6]
   (with *Hind*III recognition site underlined).

Correct size of the amplified DNA-fragment was confirmed by agarose gel electrophoresis and the DNA-fragment was purified from the gel. The DNA-fragment and the plasmid pJF119EH-*aroF-aroB* were restricted with the restriction enzyme *Hin*dIII and the linear plasmid DNA was dephosphorylated. The two DNA-fragments were ligated and used for the transformation of chemically competent cells of *E*. *coli* DH5α.

Transformants were selected on LB agar plates containing 100 mg·l⁻¹ ampicillin. A plasmid showing the correct insert sequence (as confirmed by sequencing) was called pJF119EH-*aroF-aroB-aroL.*

A 2124 bp DNA-fragment comprising the genes *entC* and *entB* for the isochorismate synthase and isochorismatase of *E. coli* was amplified by PCR from plasmid pDF3 (Franke et al. (2003) Chem.Eur.J., 9: 4188-4196) using the following primers:
5' - CCCAGATCTTTATCATTTTGTGGAGGATGA - 3' [SEQ ID: No. 7]
   (with *Bgl*I recognition site underlined) and
5' - CCCAGATCTGAAATCCATTATTTCACCTCGC - 3' [SEQ ID: No. 8]
   (with *Bgl*I recognition site underlined).

Correct size of the amplified DNA-fragment was confirmed by agarose gel electrophoresis and the DNA-fragment was purified from the gel. The DNA-fragment was restricted with the restriction enzyme *Bg*/l, the plasmid pJF119EH-*aroF-aroB-aroL* was restricted with the restriction enzyme *Bam*HI, and the linear plasmid DNA was dephosphorylated. The two DNA-fragments were subsequently ligated and used for the transformation of chemically competent cells of *E. coli* DH5α.

Transformants were selected on LB agar plates containing 100 mg·l⁻¹ ampicillin. A plasmid showing the correct insert sequence (as confirmed by sequencing) was called pC20 (= pJF119EH-*aroF-entC-entB-aroB-aroL*).

### Example 2: Construction of host strains F82 and F111

Gene inactivations in *E*. *coli* were performed according to the procedure of Datsenko and Wanner ((2000) PNAS, 97: 6640-6645) using the plasmids pKD3 and pKD4 as templates for the inactivation cassettes. The target chromosomal locus, comprising one or more genes, is first replaced by an antibiotic resistance gene. Subsequently, the resistance gene can be eliminated leaving behind a marker-less chromosomal deletion.

In order to delete the *pheA-tyrA-aroF* gene locus of *E*. *coli* W3110 strain LJ110 (T. Zeppenfeld et al., (2000) J.Bacteriol., 182: 4443-4452) the inactivation cassette was amplified by PCR from the plasmid pKD4 using the following primers:

5' - AACTTCGTCGAAGAAGTTGAAGAAGAGTAGTCCTTTATATTGAGTGTATCGT-
GTAGGCTGGAGCTGCTTC- 3' [SEQ ID: No. 9]

5' - AGTGTAAATTTATCTATACAGAGGTAAGGGTTGAAAGCGCGACTAAATTGCA-
TATGAATATCCTCCTTAG- 3' [SEQ ID: No. 10]

A strain carrying the deletion of the *pheA-tyrA-aroF* gene locus was designated F4. Due to the deletion of the genes *tyrA* (chorismate mutase/prephenate dehydrogenase) and *pheA* (chorismate mutase/prephenate dehydratase) the strain F4 is auxotrophic for the amino acids L-tyrosine and L-phenylalanine.

In order to delete additionally the *entC-entE-entB-entA* operon of the strain F4 the inactivation cassette was amplified by PCR from the plasmid pKD3 using the following primers:

5' - TAAATGATAATCATTATTAAAGCCTTTATCATTTTGTGGAGGATGATATGGTG-
TAGGCTGGAGCTGCTTC - 3' [SEQ ID: No. 11]

5' - TTATCGCTGGTGGCGTTCAGTTCGTCGAGCGTTAAATGGCGTTTCCAGATC-
ATATGAATATCCTCCTTAG- 3' [SEQ ID: No. 12]

An F4 derivative carrying the deletion of the *entC-entE-entB-entA* operon was designated F82. Transformation of chemically competent cells of the strain F82 with the plasmid pC20 resulted in the 2,3-CHD production strain F82/pC20.

In order to delete additionally the *gcd* gene of the strain F82 the inactivation cassette was amplified by PCR from the plasmid pKD3 using the following primers:

5' - AACAACGCGCACGTTGTCAGCAATCTGAGCAAGTCTTACTTCACATCATCGT-
GTAGGCTGGAGCTGCTTC - 3' [SEQ ID: No. 13]

5' - CTATAATTAATTGCTACTAAAATATTAATGAATTGAAATGGTGTCTCTTTCATA-
TGAATATCCTCCTTAG - 3' [SEQ ID: No. 14]

An F82 derivative carrying the inactivation of the gcd gene was designated F111. Transformation of chemically competent cells of the strain F111 with the plasmid pC20 resulted in the 2,3-CHD production strain F111/pC20.

### Example 3: Fermentative production of mixture of 2,3-CHD and gluconate in fed-batch bioreactor

The 2,3-CHD production of *E. coli* F82/pC20 from glucose was investigated in mineral medium. The precultivation medium consisted of MgSO₄·7H₂O (0,3 g·l⁻¹), CaCl₂·2H₂O (0,015 g·l⁻¹), KH₂PO₄ (3,0 g·l⁻¹), K₂HPO₄ (12,0 g·l⁻¹), NaCl (0,1 g·l⁻¹), (NH₄)₂SO₄ (5,0 g·l⁻¹), FeSO₄·7H₂O (0,075 g·l⁻¹), Na-citrate·3H₂0 (1,0 g·l⁻¹), thiamine·HCl (0,0125 g·l⁻¹), L-tyrosine (0,08 g·l⁻¹), and L-phenylalanine (0,08 g·l⁻¹). Additional minerals were added in the form of a trace element solution (1,0 ml·l⁻¹), which trace element solution was composed of Al₂(SO₄)₃·18H₂O (2,0 g·l⁻¹), CoCl₂·6H₂O (0,75 g·l⁻¹), CuSO₄·5H₂O (2,5 g·l⁻¹), H₃BO₃ (0,5 g·l⁻¹), MnCl₂·4H₂O (20,0 g·l⁻¹) Na₂MoO₄·2H₂O (3,0 g·l⁻¹), NiSO₄·6H₂O (20 g·l⁻¹), ZnSO₄·7H₂O (15,0 g·l⁻¹). A glucose stock solution (500 g·l⁻¹) was autoclaved separately and added to the sterilized medium to a final concentration of 5 g·l⁻¹.

The stock culture of *E. coli* F82/pC20 was stored at -80°C in Luria-Bertani (LB) medium containing 50% glycerol. 1 ml feedstock was used to inoculate 200 ml of precultivation medium containing ampicillin (100 mg·l⁻¹) split in two 1 I shaking flasks and incubated at 37°C and 180 rpm for 16 hours.

The fermentation medium was the same as for precultivation except for the following changes: MgSO₄·7H₂O (3,0 g·l⁻¹), K₂HPO₄ was omitted, FeSO₄·7H₂O (0,1125 g·l⁻¹), Na-citrate·3H₂0 (1,5 g·l⁻¹), thiamine·HCl (0,075 g·l⁻¹), NaCl (1 g·l⁻¹), L-tyrosine (0,3 g·l⁻¹), L-phenylalanine (0,3 g·l⁻¹), glucose (10 g·l⁻¹) and ampicillin (0,1 g·l⁻¹) Additional minerals were added in the form of a trace element solution (1,5 ml·l⁻¹). Fermentation medium was autoclaved for 20 min. at 121°C, FeSO₄ / citrate and glucose solution were autoclaved separately. Ampicillin and thiamin solutions were sterilized via filtration.

Fed-batch cultivation was performed in 7,5 I Bioengineering bioreactor (Bioengineering AG, Wald, Switzerland) with 10% inoculation for 35 h; 3,5 I initial volume, cultivation temperature 37°C, pH 6,5. pH was controlled by 12,5 % ammonia and 5 N KOH titration. After 7 h a feed for L-tyrosine and L-phenylalanine (25 g·l⁻¹ L-tyrosine, 22,8 g·l⁻¹ L-phenylalanine dissolved in 10% ammonia) was started. The optimal amino acid feed rate during the growth phase was calculated using the factor m = 2 for the volumetric L-tyrosine consumption rate (described in: Gerigk et al., Biotechnology and Bioengineering 80 (7): 746-754, 2002). After 13 h the amino acid feed rate was reduced to 2 g/h. After 5 h on-line control of the glucose concentration (OLGA. IBA GmbH, Germany) was started with a setpoint of 3 g·l⁻¹ and a glucose monohydrate feed solution (770 g·l⁻¹). After 7 h at biomass concentration (dry cell weight) of 2,2 g·l⁻¹ production was induced by addition of 0,1 mM IPTG. A total biomass concentration of 23,5 g·l⁻¹ was achieved.

A sample of the culture supernatant was lyophilized and re-dissolved in D₂O. 600 MHz ¹H-NMR at 303 K showed the resonance spectra of 2,3-CHD and gluconate. The assignment of all the resonances was done by using several 2D NMR techniques (¹H-¹H COSY, ¹H-¹H TOCSY, ¹H-¹³C COSY and ¹H-¹³C long range HMBC). All the spectra confirmed the presence of 2,3-CHD and gluconate. The amount of 2,3-CHD and gluconate present were determined to be 18,5 g·l⁻¹ and 37,8 g·l⁻¹, respectively. As a minor by-product acetate was present in concentration of 3,5 g·l⁻¹ as determined by HPLC analysis.

### Example 4: Fermentative production of gluconate-free 2,3-CHD in fed-batch bioreactor

The precultivation of the 2,3-CHD production strain F111/pC20 was performed as described in example 3.

Fed-batch cultivation was performed in 7,5 l Bioengineering bioreactor (Bioengineering AG, Wald, Switzerland) with 10% inoculation for 51 h; 3,5 l initial volume, cultivation temperature 37°C, pH 6,5. pH was controlled by 12,5 % ammonia and 5 N KOH titration. After 6 h a feed for L-tyrosine and L-phenylalanine (25 g·l⁻¹ L-tyrosine, 22,8 g·l⁻¹ L-phenylalanine dissolved in 10% ammonia) was started. The optimal amino acid feed rate during the growth phase was calculated using the factor m = 2 for the volumetric L-tyrosine consumption rate (described in: Gerigk et al., Biotechnology and Bioengineering 80 (7): 746-754, 2002). After 12 h the amino acid feed rate was reduced to 2 g/h. After 5 h on-line control of the glucose concentration (OLGA, IBA GmbH, Germany) was started with a setpoint of 3 g·l⁻¹ and a glucose monohydrate feed solution (770 g·l⁻¹). After 6 h at biomass concentration (dry cell weight) of 1,7 g·l⁻¹ production was induced by addition of 0,1 mM IPTG. A total biomass concentration of 22,4 g·l⁻¹ was achieved.

The supernatant was analyzed for 2,3-CHD by ¹H-NMR and the amount present was determined to be 21,9 g·l⁻¹. Gluconate could not be detected. Besides said amount of 2,3-CHD, acetate was found to be present in concentration of 1,1 g·l⁻¹ as determined by HPLC analysis.

### Example 5: Use of supernatant of process for the biosynthetic production of [5S,6S]-5,6-dihydroxy-cyclohexa-1,3-diene-1-carboxylic acid (2,3-trans-CHD) as a source of a co-factor for PQQ dependent enzymes

By using the glucose dehydrogenase reaction leading to the formation of gluconate in the culture supernatant of resting cells of *E*. *coli* LJ110 as model reaction, the inventors have demonstrated, that the supernatant of the process for the biosynthetic production of [5*S*,6*S*]-5,6-dihydroxy-cyclohexa-1,3-diene-1-carboxylic acid (2,3-*trans*-CHD) can be used as a source of a co-factor for PQQ dependent enzymes. In the Comparative Example 5.A, instead of 2,3-*trans*-CHD process, pure PQQ was used. In Comparative Example 5.B neither PQQ, nor any supernatant of 2,3-CHD fermentation was used.

A single colony of the *E. coli* LJ110 was used to inoculate 100 ml of LB medium. Cells were grown at 30°C with 180 rpm for 15h. At OD₅₄₆ₙₘ 4.7, the cells were harvested. After washing the cells with 50 mM potassium phosphate buffer, pH 6.5, the cells were resuspended in 8 ml of the same buffer (approx. OD₅₄₆ₙₘ 59) for the determination of gluconate formation as measure of glucose dehydrogenase activity in whole resting cells.

The test was performed in 24 well microtiter plates at 30°C at 100 rpm. Four different vials were filled with the following compounds:
1: 350 µl culture supernatant of example 3, 25 µl MgSO₄
2: 350 µl culture supernatant of example 4, 25 µl MgSO₄
A: 15 µl 50 µM PQQ, 25 µl MgS04, 335 µl 50 mM potassium phosphate buffer, pH 6.5
B: 350 µl 50 mM potassium phosphate buffer, pH 6.5, 25 µl MgSO₄.
They were used in respectively Examples 5.1 and 5.2, and in Comparative Examples 5.A and 5. B.

By addition of 100 µl of the cell suspension together with 25 µl of 1 M glucose, the reaction was started. Samples of 150 µl were taken for the gluconate determination directly after the start of the reaction and after 22 h of incubation.

Determination of the amount of gluconate was performed enzymatically with a coupled reaction of gluconate kinase and phosphogluconate dehydrogenase according to Moellering, H. & Bergmeyer, H.U., D-gluconate (D-glucono-d-lactone) and D-gluconate 6-phosphate, as described in Methods of Enzymatic Analysis (Bergmeyer, H.U. ed.), 3rd ed., Vol VI, pp.220-227, VCH Weinheim, Germany (1988), with 1:100 diluted samples. The results are given in table 1.

**Table 1**

| **Reaction** | **Presence of** | **gluconate [mM]** | | |
|---|---|---|---|---|
| | | **0 h** | **22 h** | **delta (22 h - 0h)** |
| Ex. 5.1 | 2,3 CHD fermentation supernatant of strain F82/pC22containing gluconate (example 3) | 130 | 159 | 29 |
| Ex. 5.2 | 2,3 CHD fermentation gluconate free supernatant of strain F111/pC20 (example 4) | 0 | 11 | 11 |
| Comp. Ex. 5.A | PQQ | 0 | 41 | 41 |
| Comp. Ex. 5.B | None (i.e. neither PQQ nor 2,3 CHD fermentation supernatant) | 0 | 0 | 0 |

It can be seen from these results, that the supernatant of the 2,3-CHD fermentation is able to cause conversion of glucose into gluconate in a similar way as can be achieved with PQQ as a co-factor, but at some lower activity.

All sequences of nucleotides referred to in the experimental part are listed hereinafter, and will be submitted in electronic format (Patentin).

### Annex to the application documents - subsequently filed sequences listing

Sequence listings of oligonucleotides

[SEQ ID: No.1]
CTATCGAATT CGAGCATAAA CAGGATCGCC 30

[SEQ ID: No.2]
CACTTCAGCA ACCAGTTCCC GGGGCTTCGC 30

[SEQ ID: No.3]
ACTCGTCTGC GGGATCCGTA ATTAAGGTGG 30

[SEQ ID: No.4]
AGCGGCCTAA GCTTTCTTGT TGTTACGCTG 30

[SEQ ID: No.5]
CATGACACCG GCTTTCGCCG CATAAGCTTC TATTGG 36

[SEQ ID: No.6]
TGAACGTTAA AAGCTTGCGC TCGAAAATCA ACAATT 36

[SEQ ID: No.7]
CCCAGATCTT TATCATTTTG TGGAGGATGA 30

[SEQ ID: No.8]
CCCAGATCTG AAATCCATTA TTTCACCTCG C 31

[SEQ ID: No.9]
AACTTCGTCG AAGAAGTTGA AGAAGAGTAG TCCTTTATAT TGAGTGTATC GTGTAGGCTG
GAGCTGCTTC 70

[SEQ ID: No.10]
AGTGTAAATT TATCTATACA GAGGTAAGGG TTGAAAGCGC GACTAAATTG CATATGAATA
TCCTCCTTAG 70

[SEQ ID: No.11]
TAAATGATAA TCATTATTAA AGCCTTTATC ATTTTGTGGA GGATGATATG GTGTAGGCTG
GAGCTGCTTC 70

[SEQ ID: No.12]
TTATCGCTGG TGGCGTTCAG TTCGTCGAGC GTTAAATGGC GTTTCCAGAT CATATGAATA
TCCTCCTTAG 70

[SEQ ID: No.13]
AACAACGCGC ACGTTGTCAG CAATCTGAGC AAGTCTTACT TCACATCATC GTGTAGGCTG
GAGCTGCTTC 70

[SEQ ID: No.14]
CTATAATTAA TTGCTACTAA AATATTAATG AATTGAAATG GTGTCTCTTT CATATGAATA
TCCTCCTTAG 70

## Claims

1. Process for the biosynthetic production of [5*S*,6*S*]-5,6-dihydroxy-cyclohexa-1,3-diene-1-carboxylic acid (2,3-*trans*-CHD) in an *Escherichia* microorganism having increased activity of isochorismate synthase and isochorismatase, and being deficient in activity of 2,3-dihydroxybenzoate synthase,
**characterized in that**
the process is carried out in a chorismate overproducing host microorganism also having decreased glucose dehydrogenase activity.

2. Process according to claim 1.
**characterized in that**
the decreased glucose dehydrogenase activity in the *Escherichia* microorganism has been achieved by lowering the expression of the glucose dehydrogenase gene gcd in the said microorganism.

3. Process according to any of claims 1 or 2,
**characterized in that**
the decreased glucose dehydrogenase activity in the *Escherichia* microorganism has been achieved by deletion of the glucose dehydrogenase gene gcd from the said microorganism, or by switching-off the activation of said gene by any means known to the skilled man, so as to obtain a *gcd* negative microorganism.

4. Process according to any of claims 1 to 3,
**characterized in that**
the increased activity of isochorismate synthase and isochorismatase is obtained by overexpression of at least one of the genes *entB* and *entC* and/or of the genes *entB* and *menF* from *Escherichia,* preferably from *Escherichia coli.*

5. Process according to any of claims 1 to 4,
**characterized in that**
the *Escherichia* microorganism further contains one or more overexpressed genes from the group of *aroFBL* genes from an *Escherichia* strain.

6. Process according to any of claims 1 to 5,
**characterized in that**
the *Escherichia* microorganism is an *Escherichia coli* microorganism.

7. Process according to any of claims 1 to 6,
**characterized in that**
the [5S,6S]-5,6-dihydroxy-cyclohexa-1,3-diene-1-carboxylic acid (2,3-*trans*-CHD) formed in the process is separated from the microorganism biomass by *in situ* product recovery (ISPR).

8. Use of the supernatant of a process for the biosynthetic production of [5S,6S]-5,6-dihydroxy-cyclohexa-1,3-diene-1-carboxylic acid (2,3-*trans*-CHD) where the concentration of 2,3-*trans*-CHD in the process has arrived at a concentration of at least 12 g/l as a source for a co-factor for PQQ dependent enzymes.


SEQUENCE LISTING

<110> DSM Biotech GmbH
Forschungszentrum Jülich GmbH
DSM IP Assets BV

<120> Improved biosynthetic production of [5S,6S]-5,6-dihydroxycyclohexa
-1,3-diene-1-carboxylic acid (2,3-trans-CHD)

<130> 24524EPP0

<160> 14

<170> PatentIn version 3.1

<210> 1
<211> 30
<212> DNA
<213> Artificial sequence

<220>
<223> primer

<400> 1
ctatcgaatt cgagcataaa caggatcgcc 30

<210> 2
<211> 30
<212> DNA
<213> Artificial sequence

<220>
<223> primer

<400> 2
cacttcagca accagttccc ggggcttcgc 30

<210> 3
<211> 30
<212> DNA
<213> Artificial sequence

<220>
<223> primer

<400> 3
actcgtctgc gggatccgta attaaggtgg 30

<210> 4
<211> 30
<212> DNA
<213> Artificial sequence

<220>

<223>  primer

<400>  4
agcggcctaa gctttcttgt tgttacgctg                                        30


<210>  5
<211>  36
<212>  DNA
<213>  Artificial sequence

<220>
<223>  primer

<400>  5
catgacaccg gctttcgccg cataagcttc tattgg                                 36


<210>  6
<211>  36
<212>  DNA
<213>  Artificial sequence

<220>
<223>  primer

<400>  6
tgaacgttaa aagcttgcgc tcgaaaatca acaatt                                 36


<210>  7
<211>  30
<212>  DNA
<213>  Artificial sequence

<220>
<223>  primer

<400>  7
cccagatctt tatcattttg tggaggatga                                        30


<210>  8
<211>  31
<212>  DNA
<213>  Artificial sequence

<220>
<223>  primer

<400>  8
cccagatctg aaatccatta tttcacctcg c                                      31


<210>  9
<211>  70
<212>  DNA

<213>  Artificial sequence

<220>
<223>  primer

<400>  9
aacttcgtcg aagaagttga agaagagtag tcctttatat tgagtgtatc gtgtaggctg      60

gagctgcttc                                                              70


<210>  10
<211>  70
<212>  DNA
<213>  Artificial sequence

<220>
<223>  primer

<400>  10
agtgtaaatt tatctataca gaggtaaggg ttgaaagcgc gactaaattg catatgaata      60

tcctccttag                                                              70


<210>  11
<211>  70
<212>  DNA
<213>  Artificial sequence

<220>
<223>  primer

<400>  11
taaatgataa tcattattaa agcctttatc attttgtgga ggatgatatg gtgtaggctg      60

gagctgcttc                                                              70


<210>  12
<211>  70
<212>  DNA
<213>  Artificial sequence

<220>
<223>  primer

<400>  12
ttatcgctgg tggcgttcag ttcgtcgagc gttaaatggc gtttccagat catatgaata      60

tcctccttag                                                              70


<210>  13
<211>  70
<212>  DNA
<213>  Artificial sequence

<220>
<223>  primer

<400>  13
aacaacgcgc acgttgtcag caatctgagc aagtcttact tcacatcatc gtgtaggctg      60

gagctgcttc                                                              70


<210>  14
<211>  70
<212>  DNA
<213>  Artificial sequence

<220>
<223>  primer

<400>  14
ctataattaa ttgctactaa aatattaatg aattgaaatg gtgtctcttt catatgaata      60

tcctccttag                                                              70